# EUROPEAN PATENT APPLICATION

(11) **EP 0 909 558 A2**
(43) Date of publication of application: **21.04.1999**
(21) Application number: 98115893.4
(22) Date of filing: 24.08.1998
(51) Int. Cl.: A61K 9/00, A61K 31/415

(54) **Chroman derivative containing ophthalmic solution**

(30) Priority: 29.08.1997 JP 233990/97
(71) Applicant: SANTEN PHARMACEUTICAL CO., LTD., Higashiyodogawa-ku, Osaka-shi, Osaka 533-8651 (JP); SANWA KAGAKU KENKYUSHO CO., LTD., Higashi-ku Nagoya-shi Aichi-ken 461 (JP)
(72) Inventor: Ota, Atsutoshi c/o Santen Pharmaceutical Co., Ltd, Ikoma-shi Nara 630-01 (JP); Kuwano, Mitsuaki c/o Santen Pharmaceutical Co.,LTD, Ikoma-shi Nara 630-01 (JP); Takada, Koichi c/o Santen Pharmaceutical Co.,LTD., Ikoma-shi Nara 630-01 (JP); Hibi, Chihiro c/o Sanwa Kagaku Kenkyusho Co.,Ltd., Nagoya-shi Aichi 461 (JP)
(74) Representative: Paul, Dieter-Alfred, Dipl.-Ing.

(57) **Abstract**

The object of the present invention is to provide an ophthalmic solution with improvement in the ocular penetration of (2S, 4S)-6-fluoro-2',5'-dioxospiro[chroman-4,4'-imidazolidine]-2-carboxamide as an active ingredient. The ophthalmic solution of the present invention comprises (2S, 4S)-6-fluoro-2',5'-dioxospiro[chroman-4,4'-imidazolidine]-2-carboxamide as an active ingredient wherein the osmotic pressure ratio is adjusted to less than 1 and the viscosity to more than 1 cP thereby ocular penetration of the active ingredient are remarkably improved. As a viscosity increasing agent, a cellulose-based polymer for example is formulated. The cellulose-based polymer is preferably hydroxypropylmethylcellulose.

## Description

### BACKGROUND OF THE INVENTION

The present invention relates to an ophthalmic solution containing (2S, 4S)-6-fluoro-2',5'-dioxospiro[chroman-4,4'-imidazolidine]-2-carboxamide as an active ingredient shown in the following formula, useful for treatment of diabetic keratopathy etc.

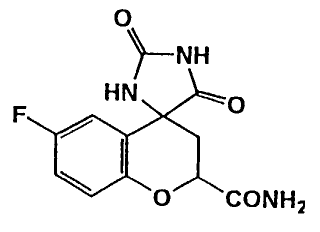

Japanese Patent Publication No. 72227/1991 reports (2S, 4S)-6-fluoro-2',5'-dioxospiro[chroman-4,4'-imidazolidine]-2-carboxamide as a drug useful for treatment of diabetic complications, as well as a process for preparing the compound. Application thereof to ophthalmic solutions (Japanese Laid-Open Patent Publication No. 271291/1991) and use thereof as an agent for treatment of diabetic keratopathy etc. (Japanese Laid-Open Patent Publication No. 231549/1996) are also reported.

To apply a drug to ophthalmic solutions, there are various problems such as drug solubility, stability of ophthalmic solutions after preparation etc. which must be studied. The most important problem to study is to determine whether the drug can be penetrated efficiently into eyes in order to exhibit its desired pharmaceutical effect. The reason why the research and development of ophthalmic solutions are not easy is that the most appropriate formulation for each drug must be found because each drug serving as an active ingredient has different properties. As described above, there are some reports on application, to ophthalmic solutions, of (2S, 4S)-6-fluoro-2',5'-dioxospiro[chroman-4,4'-imidazolidine]-2-carboxamide which is an useful compound as an agent for treatment of diabetic keratopathy etc., but these ophthalmic solutions are still not satisfactory in view of their ocular penetration.

### SUMMARY OF THE INVENTION

As a result of their precise study on improvement in ocular penetration of (2S, 4S)-6-fluoro-2',5'-dioxospiro[chroman-4,4'-imidazolidine]-2-carboxamide (referred to hereinafter as the present compound), the present inventors found that the objective ophthalmic solution can be obtained by allowing the solution to have a low osmotic pressure, that is, an osmotic pressure ratio of less than 1 and further to have an increased viscosity, that is, a viscosity is more than 1 centipoise (cP).

### DETAILED DESCRIPTION

The osmotic pressure of ophthalmic solution is affected by the amounts of the present compound and additives in the solution. In the present invention, the osmotic pressure ratio is adjusted to less than 1 by controlling the amounts of these materials affecting the osmotic pressure ratio. Specifically, adjustment of the osmotic pressure ratio is conducted mainly by controlling the amounts of additives formulated as osmotic pressure adjusting agent. Examples of such osmotic pressure adjusting agent are polyhydric alcohols such as mannitol and glycerin and salts such as sodium chloride and potassium chloride. These osmotic pressure adjusting agent can be used alone or in combination thereof.

Adjustment of the viscosity is conducted as follows. The viscosity of water is about 0.9 cP (25 °C), and if no viscosity increasing agent is used in the ophthalmic solution, their viscosity is usually 1 cP or less. In the present invention, the viscosity is adjusted to more than 1 cP by incorporating a viscosity increasing agent. The viscosity increasing agent can be any one applicable to ophthalmic solutions and is not particularly limited. Cellulose-based polymers are preferably used. Examples of such cellulose-based polymers are hydroxypropylmethylcellulose, hydroxyethylcellulose etc., among which hydroxypropylmethylcellulose (HPMC) is particularly preferred. These viscosity increasing agents can be used alone or in combination thereof.

The essence of the present invention lies in the improvement of ocular penetration of the present compound as the active ingredient by decreasing the osmotic pressure ratio of ophthalmic solutions and simultaneously by increasing the viscosity. As will be made apparent in the test of ocular penetration of the present compound described below, the ocular penetration is improved as the osmotic pressure ratio is decreased, and the ocular penetration is improved as the viscosity is increased. However, the decreasing osmotic pressure ratio brings about a raise in irritation at the time of instillation with ophthalmic solution, while the increasing viscosity causes an increase in blurring at the time of instillation with the solution. The irritation and blurring at the time of instillation with ophthalmic solution vary depending on users, thus making it difficult to determine the optimum conditions. However, by suitably adjusting the osmotic pressure ratio and viscosity, it is possible to obtain ophthalmic solutions with less irritation, less blurring, and less resistance to successive application.

From the comprehensive viewpoint of this ocular penetration and the irritation and blurring at the time of instillation with ophthalmic solution, a preferable range of the osmotic pressure ratio and viscosity can be selected for ophthalmic solutions.

Specifically, the applicable osmotic pressure ratio in the present invention is in the range of 0.1 to 0.9, preferably 0.3 to 0.6, more preferably 0.4 to 0.5. Since the osmotic pressure is affected by other additives as well, the amount of the osmotic pressure adjusting agent is suitably controlled in consideration of the amount of such additives, whereby the osmotic pressure ratio is adjusted to the desired level.

Specifically, the applicable viscosity of the ophthalmic solution of the present invention is more than 1 cP, and is preferably in the range of 2 to 50 cP, more preferably in the range of 5 to 25 cP. In the case where e.g. HPMC ("60SH4000", Shin-Estsu Chemical Industry Co., Ltd.) is used as a viscosity increasing agent, the amount thereof for adjusting the viscosity to the range of 2 to 50 cP is 0.05 to 0.75 % by weight and the amount for adjusting the viscosity to the more preferable range of 5 to 25 cP is 0.1 to 0.55 % by weight. The viscosity in the present invention refers to that determined at 25 °C with a rotational viscometer.

Although the content of the present compound in the ophthalmic solution of the present invention can be suitably selected depending on the disorders and symptoms to be treated, the content is 0.01 to 0.3 % by weight, preferably 0.05 to 0.2 % by weight in consideration of the solubility of the present compound.

In preparation of the ophthalmic solution of the present invention, a wide variety of additives can be added to the above-described indispensable ingredients, if necessary. Examples of such additives are stabilizers such as EDTA and the like, buffers such as ε-aminocaproic acid and the like, preservatives such as benzalkonium chloride and the like, pH adjusting agent etc. Because the stability of the present compound in an aqueous solution is lowered when the pH value becomes over 7, the pH value of the ophthalmic solution of the present invention is preferably adjusted to 7 or less, particularly 6 to 7. The process for preparing the ophthalmic solution of the present invention does not require any special techniques, and the solution can be prepared by any widely used processes. Specific examples of formulations of the ophthalmic solution are described in Examples below, which however are intended to aid the understanding of the present invention but not to limit the scope of the present invention.

### EXAMPLES

### 1. Test of ocular penetration of the present compound

### 1) Relationship between osmotic pressure and ocular penetration

The influence of osmotic pressure ratio on ocular penetration of the present compound was examined in the following experiment.

### (Experimental method)

Preparation of ophthalmic solution: 0.2 g of the present compound and additives were dissolved in sterile purified water, followed by adding and dissolving sodium chloride in an amount enough to achieve the desired osmotic pressure ratio. Then, the solution was adjusted with sodium hydroxide to pH 7, and the total volume was adjusted to 100 ml by adding sterile purified water. The full formulation is shown in Formulation Example 1:

### Formulation Example 1 (in 100 ml)

| | |
|---|---|
| The present compound | 0.2 g |
| Benzalkonium chloride | 0.005 g |
| ε-Aminocaproic acid | 0.1 g |
| Sodium chloride | Required amount |
| Sodium hydroxide | q. s. |
| Sterile purified water | q. s. |

Administration and measurement: The ophthalmic solution prepared above were instilled into the eyes of JW strain male white rabbits (4 eyes of 2 animals per group), and 0.5 hour after application of the solution, the concentration of the present compound in the cornea was determined by HPLC.

### (Results and Discussion)

The results (mean values) measured by HPLC are shown in Table 1.

**Table 1**

| | | | | | |
|---|---|---|---|---|---|
| Osmotic pressure ratio | 0.06 | 0.25 | 0.5 | 1.0 | 2.0 |
| Concentration of the present compound in the cornea (ng/g) | 481 | 138 | 111 | N.D | N.D |
| N.D. represents that the present compound could not be detected. | | | | | |

The above result indicates that the present compound is hardly penetrated into the cornea when the osmotic pressure ratio is more than 1, whereas the amount thereof penetrated is remarkably increased with a decreasing osmotic pressure ratio.

### 2) Relationship of viscosity and osmotic pressure ratio to ocular penetration

The influence of viscosity and osmotic pressure ratio on ocular penetration of the present compound was examined in the following experiment.

### (Experimental method)

Preparation of ophthalmic solution: 0.2 g of the present compound and additives were dissolved in sterile purified water, followed by adding and dissolving HPMC and sodium chloride in amounts enough to achieve the desired viscosity and osmotic pressure ratio. Then, the solution was adjusted with sodium hydroxide to pH 7, and the total volume was adjusted to 100 ml by adding sterile purified water. The full formulation is shown in the following Formulation Example 2:

### Formulation Example 2 (in 100 ml)

| | |
|---|---|
| The present compound | 0.2 g |
| Benzalkonium chloride | 0.005 g |
| HPMC | Required amount |
| Sodium chloride | Required amount |
| Sodium hydroxide | q. s. |
| Sterile purified water | q. s. |

Administration and measurement: The ophthalmic solution prepared above was instilled into the eyes of JW strain male white rabbits (4 eyes of 4 animals per group), and 0.5 hour after application of the solution, the concentration of the present compound in the cornea was determined by HPLC.

### (Results and Discussion)

The results (mean values) measured by HPLC are shown in Table 2.

**Table 2**

| | | | | | | | |
|---|---|---|---|---|---|---|---|
| Osmotic pressure ratio | 0.5 | 0.5 | 0.4 | 0.4 | 0.4 | 0.3 | 0.3 |
| Viscosity (cP) | 25 | 13 | 25 | 13 | 7 | 13 | 7 |
| Concentration of the present compound in the cornea (ng/g) | 505 | 329 | 447 | 456 | 251 | 616 | 265 |

These results clearly show that the amount of the compound penetrated into the cornea is increased by increasing the viscosity when the osmotic pressure ratio is the same.

### 2. Test of irritation with ophthalmic solutions

Various kinds of ophthalmic solutions with varying osmotic pressure ratio and viscosity were prepared from Formulation Example 2, and feelings (the presence or absence of irritation, blurring etc.) upon instillation of the solutions into human eyes were evaluated. The results indicated that ophthalmic solutions with the osmotic pressure ratio adjusted to 0.4 or 0.5 and with the viscosity adjusted to 7 cP or 13 cP were particularly preferable.

### 3. Preparation Examples

Specific examples of formulations of the ophthalmic solution of the present invention are as follows: Formulation Example 1 (osmotic pressure ratio of 0.4, viscosity of 15 cP, pH 6.5)

| | |
|---|---|
| The present compound | 0.2 g % by weight |
| Sodium chloride | 0.33 % by weight |
| HPMC | 0.45 % by weight |
| Benzalkonium chloride | 0.005 % by weight |
| Sodium hydroxide | q. s. |
| Hydrochloric acid | q. s. |

By varying the concentration of the present compound, sodium chloride or HPMC in Formulation Example 1, ophthalmic solutions are prepared which contain the present compound at concentrations of 0.01, 0.05, 0.1, 0.2 and 0.3 % by weight, with osmotic pressure ratios of 0.1, 0.3, 0.4, 0.5, 0.6 and 0.9 and viscosities of 2, 5, 7, 10, 13, 15, 20 and 25 cP, respectively. Formulation Example 2 (osmotic pressure ratio of 0.4, viscosity of 15 cP, pH 6.5)

| | |
|---|---|
| The present compound | 0.2 % by weight |
| ε-Aminocaproic acid | 0.5 % by weight |
| Mannitol | 1.15 % by weight |
| HPMC | 0.45 % by weight |
| Benzalkonium chloride | 0.005 % by weight |
| Sodium hydroxide | q. s. |
| Hydrochloric acid | q. s. |

By varying the concentration of the present compound, mannitol or HPMC in Formulation Example 2, ophthalmic solutions are prepared which contain the present compound at concentrations of 0.01, 0.05, 0.1, 0.2 and 0.3% by weight, with osmotic pressure ratios of 0.1, 0.3, 0.4, 0.5, 0.6 and 0.9 and viscosities of 2, 5, 7, 10, 13, 15, 20 and 25 cP, respectively.

According to the present invention, there can be provided ophthalmic solutions with improvement in the ocular penetration of (2S, 4S)-6-fluoro-2',5'-dioxospiro[chroman-4,4'-imidazolidine]-2-carboxamide as the active ingredient.

## Claims

1. An ophthalmic solution comprising (2S, 4S)-6-fluoro-2',5'-dioxospiro[chroman-4,4'-imidazolidine]-2-carboxamide as an active ingredient wherein an osmotic pressure ratio is adjusted to less than 1 and a viscosity to more than 1 cP thereby improving ocular penetration of said active ingredient.

2. The ophthalmic solution according to claim 1 wherein a cellulose-based polymer is incorporated as a viscosity increasing agent.

3. The ophthalmic solution according to claim 1 wherein the osmotic pressure ratio is from 0.3 to 0.6 and the viscosity is from 2 to 50 cP.

4. The ophthalmic solution according to claim 1 wherein the osmotic pressure ratio is from 0.4 to 0.5 and the viscosity is from 5 to 25 cP.

5. The ophthalmic solution according to claim 2 wherein the cellulose-based polymer is hydroxypropylmethylcellulose.
